# EUROPEAN PATENT APPLICATION

(11) **EP 4 623 895 A1**
(43) Date of publication of application: **01.10.2025**
(21) Application number: 24861335.8
(22) Date of filing: 27.06.2024
(51) Int. Cl.: A61K 8/73, A61Q 19/08

(54) **COMPOSITION COMPRISING HYDROLYZED COLANIC ACID SALT AND USE THEREOF IN SKIN ANTI-AGING AND SKIN PROTECTION**

(30) Priority: 09.02.2024 WO PCT/CN2024/077145
(71) Applicant: Shenzhen Pam2L Biotechnologies Co., Ltd., Shenzhen, Guangdong 518132 (CN)
(72) Inventor: ZHONG, Chao, Shenzhen, Guangdong 518132 (CN); CUI, Junfeng, Shenzhen, Guangdong 518132 (CN); PU, Jiahua, Shenzhen, Guangdong 518132 (CN)
(74) Representative: Barrow, Nicholas Martin
(86) International application number: PCT/CN2024/102087
(87) International publication number: WO 2025/166972

(57) **Abstract**

Disclosed is a composition comprising colanic acid salt and use thereof in skin anti-aging and skin protection. The colanic acid salt has good skin anti-aging and skin protection efficacy.

## Description

### TECHNICAL FIELD

The invention belongs to the field of skincare products and medicines, and specifically relates to a composition comprising hydrolyzed colanic acid salt and use thereof in skin anti-aging and skin protection.

### BACKGROUND

Colanic acid (CA) is a bacterial extracellular polysaccharide, produced by most *Escherichia coli* strains and other species in the *Enterobacteriaceae* family, which is a polysaccharide synthesized by bacteria in the course of their life activities to adapt to changes in environment and to improve their chances of survival. CA has a high molecular weight and is loosely wrapped around the surface of the bacteria, such that the bacteria exhibit a mucus state, preventing the cell from losing water and protecting the cell from harmful substances. Under unfavorable environmental conditions, such as dryness, low pressure, and low pH, mucoid strains have greater survivability than wild strains. In 2017, Han *et al.* reported the ability to significantly prolong the lifespan of *Caenorhabditis elegans* by feeding purified CA or feeding CA-secreting *E. coli.* In addition, CA, as a unique active biopolymer with special biological properties and physiological parameters, has a wide application prospect.

### SUMMARY

In one aspect, the present disclosure provides use of a colanic acid or a physiologically acceptable salt thereof, or a composition comprising a colanic acid or a physiologically acceptable salt thereof, in skin anti-aging and/or skin protection, the molecular weight of the colanic acid or the physiologically acceptable salt thereof is in a range of 1-10 kDa.

In some embodiments, the skin anti-aging comprises improving collagen content in skin, preferably type I and type III collagen content in skin.

In some embodiments, the skin protection comprises enhancing protection function of skin cells, such as increasing viability of the skin cells.

In some embodiments, the skin anti-aging and the skin protection comprise improving condition or appearance of skin.

In some embodiments, the content of the colanic acid or the physiologically acceptable salt thereof is 0.01-10% (w/v), 0.1-5% (w/v), or 0.5-3% (w/v).

In some embodiments, the physiologically acceptable salt of colonic acid is colanic acid sodium salt.

In some embodiments, the colanic acid or the physiologically acceptable salt thereof, or the composition comprising the colanic acid or the physiologically acceptable salt thereof, is formulated for topical, oral, intramuscular, subcutaneous or intravenous administration.

In some embodiments, the composition comprises the colanic acid or the physiologically acceptable salt thereof further comprises a cosmetically or pharmaceutically acceptable additive.

In another aspect, the present disclosure provides use of the colanic acid or the physiologically acceptable salt thereof, or the composition comprising the colanic acid or the physiologically acceptable salt thereof as described above in the preparation of a medicament or cosmetic product for skin anti-aging and/or skin protection.

In another aspect, the present disclosure provides a composition comprising the colanic acid or the physiologically acceptable salt thereof as an active ingredient, the molecular weight of the colanic acid or the physiologically acceptable salt thereof is in a range of 1-10 kDa.

In some embodiments, the molecular weight of the colanic acid or the physiologically acceptable salt thereof is in a range of 1-3 kDa or 3-10 kDa.

In some embodiments, the content of the colanic acid or the physiologically acceptable salt thereof in the composition is 0.01-10% (w/v), 0.1-5% (w/v), or 0.5-3% (w/v).

In some embodiments, the composition comprises merely the colanic acid or the physiologically acceptable salt thereof as the active ingredient.

In some embodiments, the composition is formulated for topical, oral, intramuscular, subcutaneous or intravenous administration.

In some embodiments, the composition is formulated for topical administration.

In some embodiments, the composition further comprises a cosmetically or pharmaceutically acceptable additive.

In some embodiments, the physiologically acceptable salt of colanic acid is colanic acid sodium salt.

### BRIEF DESCRIPTION OF THE DRAWINGS

The invention may be more fully understood with reference to the following drawings.
FIG. 1 shows the results of transcriptome analysis of small and large molecules of colanic acid sodium salt on fibroblasts.
FIG. 2 shows a multi-dimensional efficacy mechanism of the anti-aging of colanic acid.
FIG. 3 shows the results of transcriptome analysis of oligo colanic acid sodium salt on human fibroblasts.
FIG. 4 shows results of transcriptome analysis of oligo colanic acid sodium salt on human fibroblasts (A) and cell viability test results after UVA irradiation (B).
FIG. 5 shows the inhibitory effect of oligo colanic acid sodium salt on TNF-α and IL-6 of macrophages.
FIG. 6 shows the transcription regulation results of oligo colanic acid sodium salt on mitochondrial homeostasis repair related genes of human fibroblasts.
FIG. 7 shows the transcriptional analysis of oligo colanic acid sodium salt on SOD2 and GPX1/8 of HDF cells (A) and the reactive oxygen scavenging effect (B).
FIG. 8 shows transcriptional data of oligo colanic acid sodium salt on promoting HDF cells to synthesize type I collagen and up-regulating different types of collagens in HDF cells.
FIG. 9 shows the phototoxicity test results of oligo colanic acid sodium salt.
FIG. 10 shows the activity test results of oligo colanic acid on promoting HDF cells to synthesize type I collagen and type III collagen under light irradiation conditions.
FIG. 11 shows the MMP9 inhibitory effect of oligo colanic acid sodium salt on the skin model irradiated by ultraviolet light.
FIG. 12 shows the anti-inflammatory effect of oligo colanic acid sodium salt on human keratinocytes.
FIG. 13 shows the transdermal property test results of oligo colanic acid sodium salt.
FIG. 14 shows the positioning test results of oligo colanic acid sodium salt in cells.
FIG. 15 shows the cytotoxicity test result of oligo colanic acid sodium salt.
FIG. 16 shows the effect of oligo colanic acid sodium salt on improving the density of human dermis layer.
FIG. 17 shows the effect of oligo colanic acid sodium salt on improving the expression of collagen in human dermis layer.
FIG. 18 shows the effect of oligo colanic acid sodium salt on improving the expression of elastic protein in human dermis layer.
FIG. 19 shows the effect of oligo colanic acid sodium salt on improving the human skin elasticity and skin refinement.
FIG. 20 shows the effect of oligo colanic acid sodium salt on improving the human skin DEJ normalized area.
FIG. 21 shows the effect of oligo colanic acid sodium salt on improving the epidermis layer thickness of human skin.
FIG. 22 shows the effect of oligo colanic acid sodium salt on improving the skin redness of human skin.
FIG. 23 shows the effect of oligo colanic acid sodium salt on improving the content of 3D skin filaggrin (FLG).
FIG. 24 shows the effect of oligo colanic acid sodium salt on improving the content of 3D skin aquaporin 3 (AQP3).
FIG. 25 shows the effect of oligo colanic acid sodium salt on the water retention and moisturization of human skin.
FIG. 26 shows the effect of oligo colanic acid sodium salt on stimulating human fibroblasts to express type I collagen.
FIG. 27 shows the skin protection effect of oligo colanic acid sodium salt.

In the figures, * indicates P < 0.05 relative to negative control, ** indicates P < 0.01 relative to negative control, *** indicates P < 0.001 relative to negative control, ## indicates P < 0.01 relative to blank control, ### indicates P < 0.001 relative to blank control.

### DETAILED DESCRIPTION

Various different features and aspects of the present invention are discussed in more detail below.

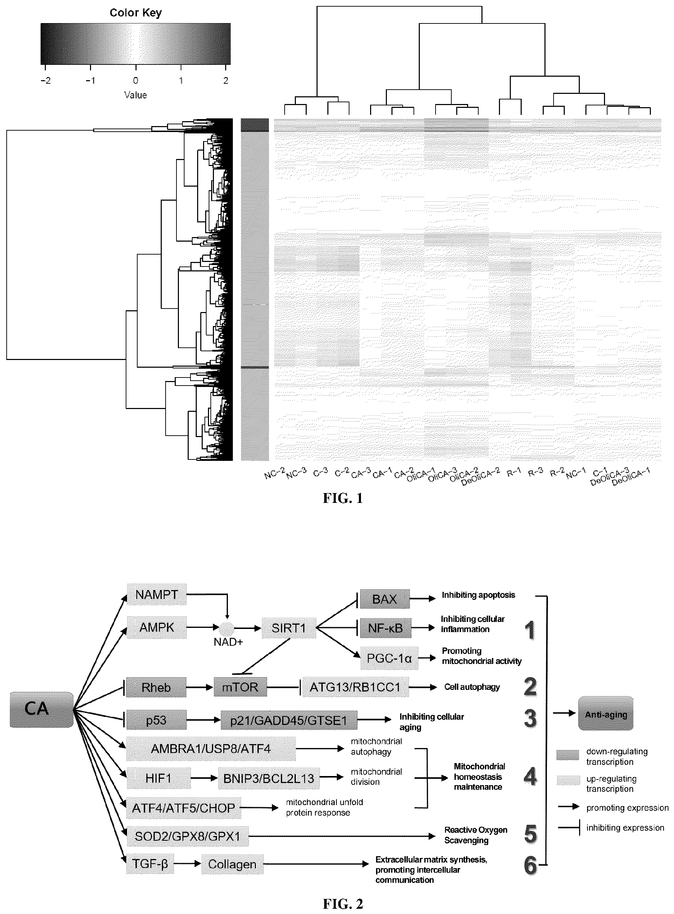

FIG. 1 shows the results of transcriptome analysis of small molecule colanic acid salt (molecular weight of 1-3 kDa, also referred to as hydrolyzed or oligo colanic acid below, abbreviated as oligo-CA or Oli-CA) and macromolecular colanic acid salt (molecular weight of 10-6000 kDa). Specifically, the salts were added to human dermal fibroblasts (HDF) cultured for 24 h, then the cells were irradiated by ultraviolet light, and continued to culture for 24 h, and then the cells were taken out for transcriptome analysis. Statistical analysis was performed on up-regulation or down-regulation of different genes according to the test results, and results show that colanic acid salt affects 2500 genes of the cells. In contrast, the control group of resveratrol only affects 426 genes. These results show that colanic acids with different molecular weights have significant effect on the transcriptome of fibroblasts.

According to the transcriptome analysis results, and in combination with KEGG long-life related pathways (https://www.kegg.jp/pathway/map04211), different anti-aging pathways of colanic acid salt are collated, and shown in FIG. 2. In FIG. 2, gray parts indicate that the transcriptome mRNA is inhibited, green parts indicate that the transcriptome mRNA is up-regulated, sharp arrows indicate promoting expression, and blunt arrows indicate inhibiting expression. FIG. 2 shows that, in the pathway 1, colanic acid salt can promote the transcriptome of NAMPT, the key enzyme for synthesizing NAD⁺ in cells, and meanwhile, colanic acid salt can also up-regulate the genes such as AMPK and sirt1, and further inhibit BAX and NF-κ, thereby inhibiting the effects of apoptosis and cell inflammation, and can also up-regulate PGC-1α to promote mitochondrial activity.

In pathway 2, colanic acid salt can inhibit Rheb and mTOR genes, thereby up-regulating expression of ATG13 and RB1CC1 gene transcription and promoting cell autophagy.

In pathway 3, colanic acid salt can promote mitochondrial autophagy by up-regulating the transcription of genes such as AMBRA1, USP8, ATF4 and the like, promote mitochondrial division by up-regulating HIF1 and promoting BNIP3 and BCL2L13 transcription, and promote mitochondrial unfold protein response by promoting the transcription of the ATF4/ATF5 and CHOP. The mitochondrial homeostasis remodeling is synergistically promoted by the three aspects above.

In pathway 4, colanic acid salt increases the activity of the cell itself to scavenge ROS by up-regulating the transcription of SOD2, GPX1, and GPX8. At the same time, in addition to scavenging reactive oxygen, the up-regulated expression of GPX8 can regulate the transfer of calcium ions from endoplasmic reticulum to mitochondria, and relieve mitochondrial damage caused by dysregulated intracellular calcium ion flow.

In pathway 5, colanic acid salt promotes the synthesis of extracellular matrix, enhances the communication between the cells, and generates better support and protection effect on cell morphology, by up-regulating the expression of collagen and various extracellular matrix transcriptome.

In the present disclosure, colanic acid (CA) or colanic acid salt can be prepared by any method as long as the desired molecular weight can be obtained. Specifically, the molecular weight of the colanic acid or the physiologically acceptable salt thereof may be 1 kDa, 2 kDa, 3 kDa, 4 kDa, 5 kDa, 6 kDa, 7 kDa, 8 kDa, 9 kDa, 10 kDa or the like, or any range between the above molecular weights. Among them, the molecular weight thereof is 1-10 kDa, 1-8 kDa, 1-6 kDa, 1-5 kDa, 1-4 kDa, 1-3 kDa, 1-2 kDa, 2-10 kDa, 2-8 kDa, 2-6 kDa, 2-4 kDa, 2-3 kDa, 3-6 kDa, 4-10 kDa, 4-8 kDa, 4-6 kDa, 5-10 kDa, 5-8 kDa, 6-10 kDa, or 6-8 kDa, which can further improve the effect of the colanic acid or the physiologically acceptable salt thereof.

The inventors of the present invention surprisingly found that the skin anti-aging and skin protection (e.g., improving skin condition or appearance, such as skin anti-aging, especially improving collagen content in the skin) efficacy can be further improved by selecting the colanic acid or the physiologically acceptable salt thereof with the above specified molecular weights.

For example, a method of CN115287314B can be employed to prepare the colanic acid or the physiologically acceptable salt thereof. In addition, the colanic acid or the physiologically acceptable salt thereof can also be purified by the method of CN114957509A. It should be understood by those skilled in the art that, by a conventional preparation method, colanic acids or colanic acid salts of different molecular weight ranges can be obtained.

The term "physiologically acceptable salt" can be any salt derived from colanic acid. In the present invention, these salts are not limited to specific species as long as they can be used in cosmetic and pharmaceutical compositions. Specific examples thereof comprise alkali metal salts such as sodium, potassium and lithium salts; alkaline earth metal salts such as calcium, magnesium, barium and zinc salts; alkylamine salts, alkylamines such as ammonia, methylamine, dimethylamine, trimethylamine, ethylamine, diethylamine, triethylamine, propylamine, butylamine, tetrabutylamine, pentylamine, and hexylamine; alkanolamine salts, alkanolamines such as ethanolamine, diethanolamine, triethanolamine, propanolamine, dipropanolamine, isopropanolamine, and diisopropanolamine; salts of other organic amines, organic amines such as piperazine and piperidine; salts of basic amino acids such as lysine, arginine, histidine, tryptophan, and the like. In the present disclosure, the physiologically acceptable salt of colanic acid can be colanic acid sodium salt.

The composition of the present invention can be used as a pharmaceutical composition or a cosmetic composition. The dosage form of the composition can be specifically selected as desired. For example, the dosage forms of the cosmetic composition can comprise emulsions, pastes, body lotions, essences, masks, gels, powders, lipsticks, cosmetic bases, foundations, lotions, ointments, patches, beauty fluid, decontamination foams, decontamination ointments, cleaning waters, soaps, or sprays. Dosage forms of pharmaceutical compositions can comprise lozenges, capsules, emulsions, dispersants, suspensions, solutions, syrups, granules, transdermal patches, gels, powders, creams, ointments, suppositories, or sprays. Pharmaceutical compositions can be formulated for topical, oral, intramuscular, subcutaneous or intravenous administration.

In the composition of the present invention, the content of the colanic acid or the physiologically acceptable salt thereof may be selected as desired, for example, can be 0.01-10% (w/v), 0.1-5% (w/v), or 0.5-3% (w/v).

In addition, as desired, the pharmaceutical composition or cosmetic composition of the present invention can contain, in addition to the essential active ingredients, additives which are normally incorporated into pharmaceutical compositions or cosmetic compositions. Examples of the additives comprise oily ingredients, moisturizers, emollients, surfactants, organic and inorganic pigments, organic powders, ultraviolet absorbers, preservatives, antibacterial agents, antioxidants, plant extracts, pH adjusters, alcohols, colorants, fragrances, blood circulation promoters, cooling agents, antiperspirants, water and the like.

In some embodiments, the composition of the present invention comprises merely the colanic acid or the physiologically acceptable salt thereof as the active ingredient. In some embodiments, the composition of the present invention further comprises other active ingredients, such as Pro-Xylane, retinol and derivatives thereof (e.g., retinyl propionate), retinene, retinoic acid, hyaluronic acid, ergothioneine, ectoine and the like.

In the present disclosure, the molecular weight of the colanic acid or the physiologically acceptable salt thereof is determined by a liquid chromatography method. The specific method is as follows: the sample and standard substance were weighed precisely, the sample was prepared into a 2 mg/mL solution, after fully dissolved, the solution was filtered through a 0.22 µm needle filter into a 1.8 mL sample injection vial. Chromatography Column: PolySep-GC-P (35*7.8 mm); PolySep-GC-P 4000 (300*7.8 mm); PolySep-GC-P 6000 (300*7.8 mm); Mobile Phase: 0.02 M NaCl solution; Flow rate: 0.6 mL/min, Column Temperature: 40 °C; Injection volume: 20 µL; Detector: Differential Detector 1260-RID.

### Preparation Method

In the following examples, oligo colanic acid sodium salt (1-10 kDa) was prepared by the following method.

The obtained colanic acid product was mixed at 37 °C for 10-12 h by adding different concentrations (2000 U/L, 3000 U/L, 5000 U/L) of colanic acid degrading enzyme (produced by PAM2LBiotechnologies, see SEQ ID No.2 in CN116334039B), the sample was collected and heated to deactivate the enzyme, then a DEAE ion column was used for separation and purification to remove a small part of the nonuniform molecular weight and impurities, and colanic acid sodium salt product with different molecular weights were obtained. The final product can be obtained by lyophilization or spray drying. The obtained colanic acid sodium salt has a molecular weight of 1-10 kDa, 1-8 kDa, 1-6 kDa, 1-5 kDa, 1-4 kDa, 1-3 kDa, 1-2 kDa, 2-10 kDa, 2-8 kDa, 2-6 kDa, 2-4 kDa, 2-3 kDa, 3-6 kDa, 4-10 kDa, 4-8 kDa, 4-6 kDa, 5-10 kDa, 5-8 kDa, 6-10 kDa, or 6-8 kDa.

Among them, the colanic acid product macromolecular colanic acid sodium salt (3000-6000 kDa) was prepared by the following method.
1. Solid-liquid separation
   1) Dilution: the fermentation broth was pumped into a storage tank, diluted 3-20 times with 10-60% calcium chloride solution (the amount of calcium chloride was 2% (w/v) of fermentation broth) and purified water, and stirred for 2-3 h until fully dissolved.
   2) Flocculation: after adding solid sodium carbonate to adjust the pH to 9.0-12.0, the mixture was flocculated for 1-2 h and then premixed with diatomite (the amount of diatomite was 1% (w/v) of fermentation broth).
   3) Plate and frame filtration: according to the operation SOP of plate and frame filter press, the filter cloth was arranged and the filter plate was pressed. The plate and frame filter press system was rinsed with 0.5 M sodium hydroxide solution for 15 min, and rinsed with purified water until the pH test paper showed neutral. The feed pump was started to pump the diatomite suspension (pre-coating amount was 0.5 kg/m²), and diatomite was pre-coated into the plate frame. After the pre-coating was finished, the liquid inlet was connected to the liquid storage tank and the flocculation liquid was pumped into the plate and frame filter for circular filtration, and the working pressure was controlled to be < 0.2 MPa. The outlet of the liquid collecting tube was connected to the feeding tank for circular filtration, after the liquid at the outlet was clear (turbidity ≤ 50 NTU), collection of clear filtering liquid was started. After the end, 1-2 times of the dead volume of purified water was eluted.
2. Activated carbon adsorption
   1) Adsorption: Injection-grade activated carbon was added at an amount of 0.5% of the weight of fermentation broth, pH was adjusted to 4-6 with 0.1 M HCl, and stirring was performed for 1-2 h for absorption. After adsorption was completed, pre-mixed diatomite (the amount of diatomite was 0.1-5% (w/v) of fermentation broth).
   2) Plate and frame filtration: according to the operation SOP of plate and frame filter press, the filter cloth was arranged and the filter plate was pressed. The feed pump was started to pump the diatomite suspension (pre-coating amount was 0.5 kg/m²), and diatomite was pre-coated into the plate frame. After the pre-coating was finished, the liquid inlet was connected to the liquid storage tank and the flocculation liquid was pumped into the plate and frame filter for circular filtration, and the working pressure was controlled to be < 0.2 MPa. The outlet of the liquid collecting tube was connected to the feeding tank for circular filtration, after the liquid at the outlet was clear (turbidity ≤ 30 NTU), collection of clear filtering liquid was started.
      After the end, 1-2 times of the dead volume of purified water was eluted.
3. Fine filtration
   The filtrate was adjusted to pH 7.0±0.2 with solid sodium carbonate, and then the filtrate was subjected to fine filtration by using a 20-inch ultrahigh precision PP filter element in series with a PES filter element. The feed flow rate was slowly increased, such that the filtration pressure was ≤ 0.1 MPa; if the pressure was too high, the feed liquid was backflushed and emptied, and the filter was replaced to filter again. When continuous liquid flowed out of the exhaust port, the exhaust valve was closed and the filtrate was collected.
4. Ceramic membrane concentration
   1) Cleaning: the ceramic membrane ultrafiltration system was connected, and the ceramic membrane system was circular rinsed sequentially with 2% citric acid, purified water and 0.5 M NaOH solution for 15-30 min, and then was rinsed with purified water until the pH paper at the transmission end showed neutral.
   2) Concentration: the refined filtered liquid was pumped into a ceramic membrane circulating liquid storage tank, the outlet of the return end was connected to the circulating liquid storage tank, and then the tank low valve was opened. The return valve and the through valve at one end were fully opened. The system was started, the frequency was controlled to 10-80 Hz, and the feed flow rate was controlled to be 100-1500 L/h. The inlet pressure was controlled to be < 0.1 MPa by setting the alarm pressure at 0.1 MPa (reduce the feed flow rate, if overpressure). When the circular concentration was performed to 1/2 of the volume of fermentation broth or the inlet pressure was ≥ 0.1 MPa, the concentration was stopped. The through end valve was closed, the ceramic membrane system was circular rinsed with 1-5 times of the system dead volume of purified water for 5-30 min, the liquid at the return end was emptied and collected again, and the above rinsing and collection operations was repeated 2 times. The collected concentrate and rinse solution were combined.
5. Alcohol precipitation
   1) Sodium salt treatment: 10-40% sodium chloride solution (final concentration of sodium chloride was 1-5%) was added to the concentrate and rinse solution, and stirred uniformly. The fine filtration was performed by using a 20-inch ultrahigh precision PP filter element in series with a PES filter element. The feed flow rate was slowly increased, such that the filtration pressure was ≤ 0.1 MPa; if the pressure was too high, the feed liquid was backflushed and emptied, the filter was replaced to filter again, and the filtrate was collected.
   2) Ethanol precipitation: the alcohol precipitation tank was washed sequentially with citric acid, purified water and NaOH solution for 15-30 min, and then rinsed with purified water until the pH test paper showed neutral. The filtrate in the previous step was pumped into an alcohol precipitation tank, stirring was started, and 95% ethanol of 2 times of the volume of feed liquid was slowly added. Stirring until a large amount of white flocculent precipitate was produced, and then stirring was stopped. The mixture was rested for precipitation for 2-4 h, the supernatant was pumped off, and the precipitate was collected.
6. Washing and Dehydration
   1) Washing: 1-10 times of 70% ethanol (containing 1% sodium chloride) was added into the CA saccharide precipitate, the solution was stirred and washed for 20 min, and then placed for 1-8 h, the supernatant was pumped off, and the precipitate was collected. The wash was then repeated 2 times and the precipitate was collected.
   2) Dehydration: 1-10 times of the volume of 95% ethanol was added into the precipitate, the mixture was stirred and dehydrated for 2h, rested for 1-2 h, and then the supernatant was pumped off. The dehydration operation was repeated once, and the precipitate was collected. After the precipitate was subjected to vacuum suction filtration to reduce residual liquid, 1-10 times of the volume of 95% ethanol was added again for dehydration operation. The precipitate was collected, the liquid was suction-filtered again, and the wet weight of the sample was weighed.
7. Drying
   The wet weight sample was placed in a vacuum drying oven (the sample thickness was not more than 2 cm), the drying temperature was set to 45 °C, and the drying time was 15 h. The material was turned over every 4 hours.
8. Crushing and sieving
   After the second drying was finished, the sample was crushed again with a crushing machine, sieved through a 100-mesh sieve, and packed into aluminum foil bags.

### Experimental Method

### (1) Cytotoxicity

Cell Culture: HDF cells were cultured in DMEM complete medium (DMEM + 10% FBS + 1% P/S), placed in an incubator for static culturing at 37 °C, 5% CO₂.

Sample Treatment: CA powder was dissolved in the culture medium, and the solution was filtered and sterilized with a 0.2 µm sterile filter membrane after complete dissolving.

Experimental Treatment: HDF cells were inoculated into a 96-well plate, and a sample group, a blank control (BC), and a positive control group (PC) were set. At least 3 duplicate wells were set for each tested indicator per sample. After culturing for 24 h, the culture solution was discarded, the sample group was added with the corresponding CA solution, the positive control group was added with 10% DMSO solution, and the blank control group was added with the complete culture medium. The culturing was continued for 24 h.

Cell Viability Detection: the supernatant was discarded, complete culture medium containing 10% AmargarBlue was added, and the plate was placed in an incubator for incubation for 4 h, and the absorption value at ex/em = 560/590 nm was detected by a microplate reader.

### (2) Light Damage

Cell Culture: HDF cells were cultured in DMEM complete medium (DMEM + 10% FBS + 1% P/S), placed in an incubator for static culturing at 37 °C, 5% CO₂.

Sample Treatment: CA powder was dissolved in the culture medium, and the solution was filtered and sterilized with a 0.2 µm sterile filter membrane after complete dissolving.

Experimental Treatment: HDF cells were inoculated into a 96-well plate, a sample group, a blank control group (BC), a positive control group (PC), and a negative control group (NC) were set. At least 3 duplicate wells were set for each tested indicator per sample. After culturing for 24 h, the culture solution was discarded, the sample group was added with the corresponding CA solution, the positive control group was added with VC solution (dissolved in medium), and the negative control group and blank control group were added with the complete culture medium. The plate was incubated in an incubator for 2 h. The sample group, positive control group and negative control group were placed under a UVA lamp and irradiated with an irradiation amount of 5 J/cm². After the irradiation was completed, the culturing was continued for 24 h.

Cell Viability Detection: The supernatant was discarded, complete culture medium containing 10% AmargarBlue was added, and the plate was placed in an incubator for incubation for 4 h, and the absorption value at ex/em = 560/590 was detected by a microplate reader.

Type I Collagen Detection: The supernatant in each well was collected, and a test was performed according to the instructions of ELISA collagen detection kit (Cusabio, CSB-E08082h).

### (3) Anti-Wrinkle (Collagen Promoting)

Cell Culture: HDF cells were cultured in DMEM complete medium (DMEM + 10% FBS + 1% P/S), placed in an incubator for static culturing at 37 °C, 5% CO₂.

Sample Treatment: CA powder was dissolved in the culture medium, and the solution was filtered and sterilized with a 0.2 µm sterile filter membrane after complete dissolving.

Experimental Treatment: HDF cells were inoculated into a 96-well plate, and a sample group, a blank control group (BC), and a positive control group (PC) were set. At least 3 duplicate wells were set for each tested indicator per sample. After culturing for 24 h, the culture solution was discarded, the sample group was added with the corresponding CA solution, the positive control group was added with TGF-β solution, and the negative control group and blank group were added with the complete culture medium. The culturing was continued for 24 h.

Type I, type III Collagen Detection: The Supernatant in each well was collected, and a test was performed according to the instructions of ELISA collagen detection kit (Cusabio, CSB-E04799h).

### (4) Inflammation (Macrophages)

Cell Culture: RAW264.7 cells were cultured in DMEM complete medium (DMEM + 10% FBS + 1% P/S), placed in an incubator for static culturing at 37 °C, 5% CO₂.

Sample Treatment: CA powder was dissolved in the culture medium, and the solution was filtered and sterilized with a 0.2 µm sterile filter membrane after complete dissolving.

Experimental Treatment: RAW264.7 cells were inoculated into a 96-well plate, and a sample group, a blank control group, a positive control group and a negative control group were set. At least 3 duplicate wells were set for each tested indicator per sample. After culturing for 24 h, the culture solution was discarded, the sample group was added with the corresponding CA solution, the positive control group was added with complete culture medium containing 100 g/mL dexamethasone, and the negative control group and blank group were added with the complete culture medium. After incubation for 2 h in the incubator, 1 µg/mL of LPS (lipopolysaccharide) was added to the sample group, positive control group, and negative control group. The culturing was continued for 24 h.

Inflammation Factor Detection: The supernatants of each well were collected, and a test was performed according to the instructions of ELISA kits (Cusabio, CSB-E04740h, CSB-E08053h, CSB-E04638h).

Reactive Oxygen ROS Detection: After the supernatant was collected, cells in each well were washed twice with PBS, and a test was performed according to the instructions of the DCFH-DA kit.

### (5) Inflammation (Keratinocytes)

Cell Culture: Keratinocytes HaCaT were cultured in DMEM complete medium (DMEM + 10% FBS + 1% P/S), placed in an incubator for static culturing at 37 °C, 5% CO₂.

Sample Treatment: CA powder was dissolved in the culture medium, and the solution was filtered and sterilized with a 0.2 µm sterile filter membrane after complete dissolving.

Experimental Treatment: Cells were inoculated into a 96-well plate, and a sample group, a blank control group, a positive control group and a negative control group were set. At least 3 duplicate wells were set for each tested indicator per sample. After culturing for 24 h, the culture solution was discarded, the sample group was added with the corresponding CA sample solution, the positive control group was added with the corresponding VC solution, and the negative control group and blank control group were added with the complete culture medium. After incubation in the incubator for 2 h, a hydrogen peroxide solution (0.8 mM HX0640 Sigma-Aldrich) was added to the sample group, positive control group, and negative control group for inflammatory stimulation. The culturing was continued for 24 h.

Gene Detection: Old liquid was aspirated and discarded, the plate was washed twice with PBS, cell lysis solution was added to each well, and after pipetting to lyse the cells, samples were collected. RNA was extracted, reverse transcription was carried out to obtain cDNA, fluorescence quantitative PCR detection was carried out, and a 2-ΔΔCT method was adopted for calculation. The difference of expression amounts of the target gene in the two groups of samples was obtained by the calculated the 2-ΔΔCt value.

### (6) Transdermal Experiments

### Ex Vivo Skin Preparation

*Ex vivo* skin stored at -20 °C was thawed at room temperature with deionized water, and washed repeatedly with PBS buffer.

### Transdermal Absorption Capacity Determination

1) The *ex vivo* skin was fixed between the supply chamber and the receiving chamber of the Franz cell diffusion cell, with the skin stratum corneum side of the skin facing the supply chamber, and the dermis layer side facing the receiving chamber;
2) The receiving solution was added into the receiving chamber; after the skin was tightened and fixed, a receiving solution (PBS) was added into the receiving chamber through a sampler, and the air was exhausted to make the dermis layer of the skin in close contact with the receiving solution.
3) Sample Loading: Sample was added to the skin surface in the supply chamber. The sample was spread evenly from the central portion of the skin to the edge;
4) Penetration: The Electromagnetic stirrer was turned on to stir at a speed of 300 rpm, and (32 ± 1) °C constant-temperature water bath was maintained;
5) The skin samples were collected at time points of 1 h, 6 h and 18 h, the surface of the skin was washed with PBS, the residual liquid on the surface was wiped, and the skin was cut with a blade in circle. Sections were frozen for use.

### Observation Analysis

The skin slices were observed with a fluorescence microscope, photographed and recorded, and the fluorescence intensity was analyzed by Image J.

### (7) In Vitro Anti-Wrinkle Experiment

1) Cells Inoculation: Fibroblasts were inoculated into a 6-well plate at an inoculum density of 2.2×10⁵ cells/well, and the plate was incubated overnight in incubator (37 °C, 5% CO₂).
2) Administration: According to the test group, when the cell laying rate in the 6-well plate reached 40-60%, group administration was performed, with 2 mL of sample added to each well, and 3 duplicates wells were set in each group. After the administration was complete, the 6-well plate was placed in an incubator (37 °C, 5% CO₂) for 24 h.
3) UVA Irradiation: According to the test group, 30 J/c UVA irradiation was performed on the group which needs UVA irradiation, and then the plate was placed in an incubator (37 °C, 5% CO₂) to continue incubation for 24 h.
4) Sample Collection: After culturing for 24 h, cell culture supernatant was collected into EP tubes and placed in a -80 °C refrigerator for cryopreservation.
5) ELISA Detection: The detection was performed according to the instructions of the ELISA kit (Cusabio, CSB-E08082h).

### (8) Anti-Wrinkle and Collagen Promoting Under Light Irradiation Conditions

Cell culture: HDF cells were cultured in DMEM complete medium (DMEM + 10% FBS + 1% P/S), placed in an incubator for static culturing at 37 °C, 5% CO₂.

Sample treatment: CA powder was dissolved in the culture medium, and the solution was filtered and sterilized with a 0.2 µm sterile filter membrane after complete dissolving.

Experimental treatment: HDF cells were inoculated into a 96-well plate, and a sample group, a blank control group and a positive control group were set. At least 3 duplicate wells were set for each tested indicator per sample. After culturing for 24h, the culture solution was discarded, the sample group was added with corresponding CA solution, the positive control group was added with TGF-β solution, and the negative control group and blank group were added with complete culture medium. The sample group, positive control group and negative control group were placed under a UVA lamp and irradiated at a power of 40 mW for 4 min. After irradiation was completed, the culturing was continued for 24 h.

Type I, type III collagen detection: the supernatant of each well was collected, and the test was performed according to the instructions of an ELISA collagen detection kit (Cusabio, CSB-E04799h).

### (9) Cell Viability - CCK8 Detection

Plate inoculation: keratinocytes HaCaT were resuscitated and cultured for 1-2 days to a confluency of 70%, and after trypsin digestion, keratinocytes were inoculated into the plate at 5×10⁴ /well, and grouped as DMEM, H₂O₂-DMEM, H₂O₂-CA.

H₂O₂ stimulation: The use concentration of H₂O₂ (Shanghai Lingfeng Chemical Reagent Co., Ltd.) was 0.8 mM. First, H₂O₂ was diluted to 10 mM and then added to the culture medium at 100× (the original storage concentration was 8820 mM, 8.82 mL PBS was added with 10 µL of H₂O₂, and 40 µL of the diluent was added to 4 mL of medium). The prepared culture medium containing H₂O₂ is added into the H₂O₂ stimulation group in the form of liquid exchange and the cells were cultured for 2h.

CA treatment: 50 mg of ultraviolet-sterilized CA powder was added to 5 mL of PBS, dissolved to prepare a 10 mg/mL liquid by oscillation, and then diluted with DMEM medium to a working concentration of 5 mg/mL. The culture supernatant after H₂O₂ stimulation was removed (including DMEM blank control group), after each sample was rinsed with 100 µL of PBS, 100 µL of culture medium containing CA was added into the H₂O₂-CA group, 100 µL of culture medium was added to DMEM and H₂O₂-DMEM groups, and cultured for 24h.

CCK8 detection: the culture medium volume of the required during detection was calculated, and then CCK8 (Beyotime, C0038) was added at 10×, i.e., 400 µL of CCK8 was added to 4 mL of medium. The supernatant of each sample culture medium was aspirated, and added to a 96-well plate at 100 µL/well, and the plate was incubated for 1-2 h. The plate was detected at OD₄₅₀ using a microplate reader under dark conditions.

### EXAMPLES

The following examples are for illustrative purposes and are not intended to limit the scope or content of the present invention in any way.

In the following examples, unless otherwise specified, the colanic acid used is colanic acid sodium salt having a molecular weight of 1-3 kDa.

### Example 1

Cell Culture: Human skin fibroblast (HDF) cells were cultured in DMEM complete medium (DMEM + 10% FBS + 1% P/S), placed in an incubator for static culturing at 37 °C, 5% CO₂.

Sample Treatment: CA powder was dissolved in the culture medium, and the solution was filtered and sterilized with a 0.2 µm sterile filter membrane after complete dissolving.

Experimental Treatment: HDF cells were inoculated into a 96-well plate, and a sample group, a blank control group, a positive control group and a negative control group were set. At least 3 duplicate wells were set for each tested indicator per sample. After culturing for 24h, the culture solution was discarded, the sample group was added with corresponding CA solution, the positive group was added with VC solution (dissolved in culture medium), and the negative control group and the blank group were added with complete culture medium. The plate was incubated for 24h in an incubator. Then the cells are taken out and cryopreserved, and transcriptome analysis was carried out by GENEWIZ.

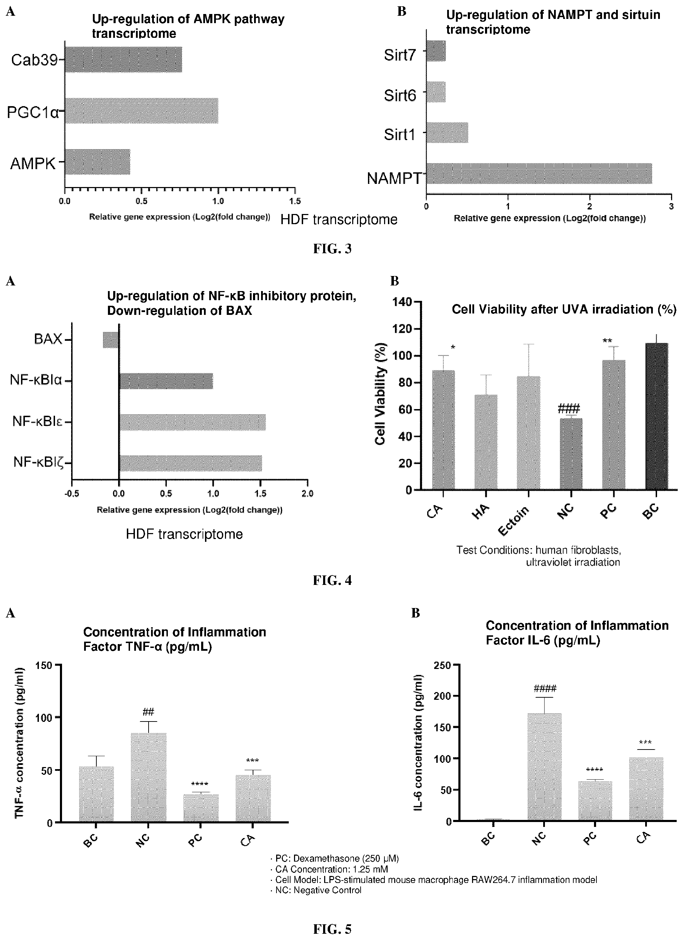

FIG. 3 shows oligo-CA (Oli-CA) promotes the transcription of AMPK, PGC1α and Cab39 (FIG. 3A), and NAMPT, sirtuin1, sirtuin6, and sirtuin7 (FIG. 3B) genes of fibroblasts under ultraviolet condition. The results in FIG. 3 show that oligo colanic acid promotes sirtuin1 expression of human fibroblasts.

### Example 2

Oligo colonic acid (2.5 mg/mL), HA (hyaluronic acid, 2.5 mg/mL), Ectoin (2.5 mg/mL), NC (ultraviolet light only), PC (resveratrol 1 mg/mL), BC (cells only) were added to fibroblasts, and then the cells were cultured for 24h, then irradiated by ultraviolet light, and cell viability rate was observed, and the effects of oligo-CA on transcription of BAX and NF-κB inhibitory protein in human fibroblasts were tested, as shown in FIG. 4. The results in FIG. 4 show that the oligo-CA inhibits BAX transcription and upregulates transcription of NF-κB inhibitory protein (FIG. 4A); after addition of oligo-CA, HA, Ectoin and Vitamin C (positive control), the cell viability after light exposure was tested. It can be seen from the above experimental results that oligo colanic acid can significantly improve the viability rate of cells under ultraviolet conditions, and has better efficacy for inhibiting apoptosis.

### Example 3

According to the method of inflammation (macrophage) in the experimental method, the inhibitory effect of oligo-CA on mouse macrophage inflammation was tested, and the results are shown in FIG. 5. As can be seen from FIG. 5, oligo colanic acid can effectively inhibit the inflammatory response of macrophages caused by LPS (lipopolysaccharide), including TNF-α (FIG. 5A), IL-6 (FIG. 5B) and the like.

### Example 4

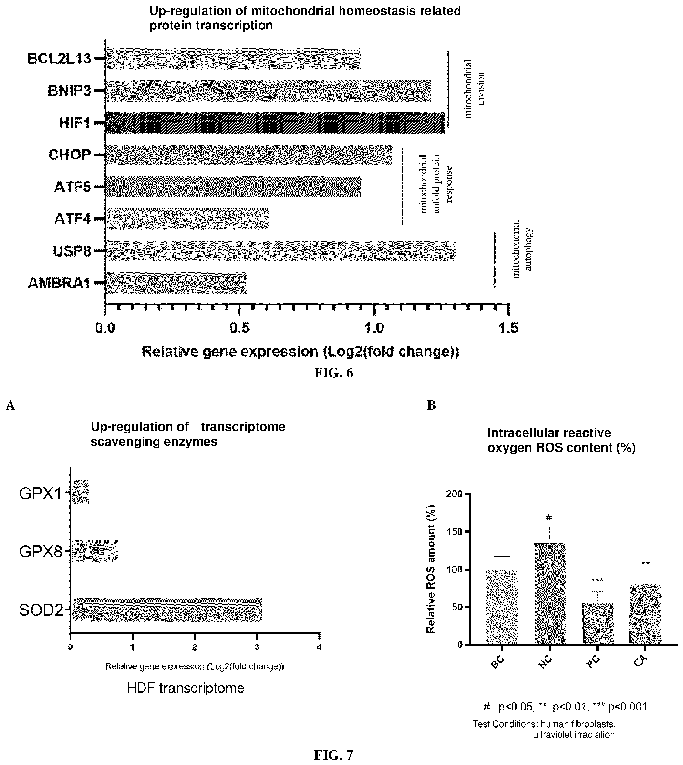

According to the experimental method of Example 1, the transcription regulation effect of oligo-CA on a human fibroblast mitochondrial homeostasis repair related genes was tested, and the results are shown in FIG. 6. FIG. 6 shows that oligo colanic acid can up-regulate the transcription of a series of genes, including AMBRA1, USP8, ATF4, BNIP3 and BCL2L13, ATF5, CHOP and the like.

### Example 5

The transcriptional analysis of oligo-CA on SOD2 and GPX1/8 of human fibroblasts and the reactive oxygen scavenging effect (Solarbio, ROS testing kit) were tested, and the results are shown in FIG. 7. It can be observed from FIG. 7 that oligo-CA can largely up-regulate the transcription levels of SOD2, GPX1 and GPX8 of human fibroblasts (FIG. 7A), while the reactive oxygen produced by the ultraviolet irradiated human fibroblasts is significantly suppressed (FIG. 7B). This indicates that oligo colanic acid has good activity in regulating the reactive oxygen scavenging of the cells.

### Example 6

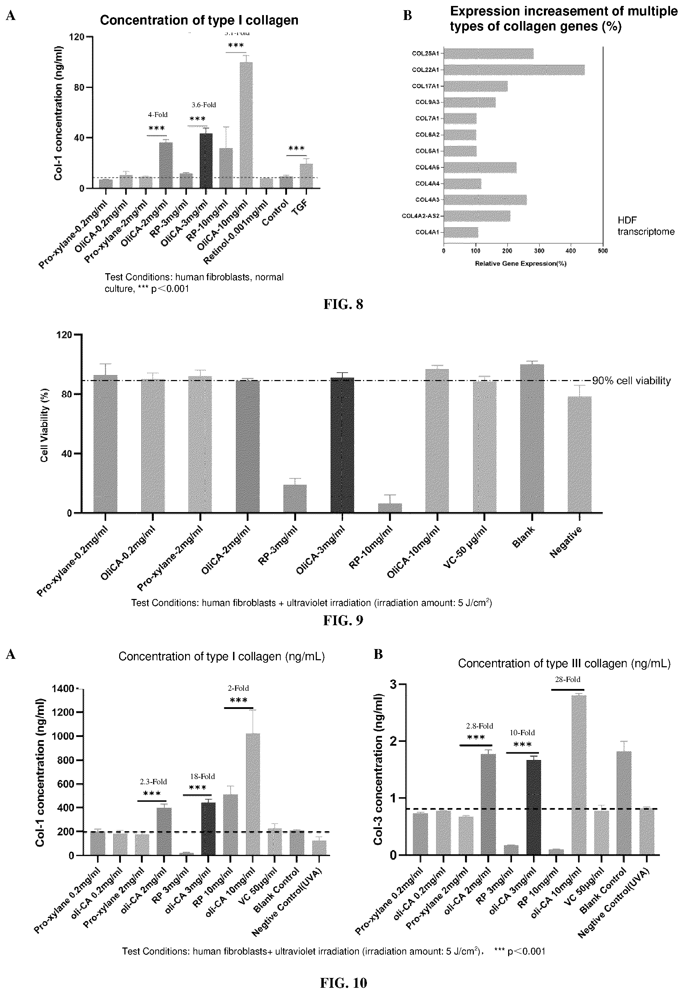

According to an experimental method for anti-wrinkles (promoting collagen) in the experimental method, data of oligo-CA on promoting type I collagen synthesis of HDF cells and up-regulating different types of collagen transcription in HDF cells were tested, and the results are shown in FIG. 8. FIG. 8 shows that oligo-CA can significantly increase the amount of type I collagen synthesized by HDF cells (the data in the figure is measured by diluting the supernatant 10-fold with the medium). It can be seen from FIG. 8A that oligo-CA has higher collagen regeneration activity than Pro-Xylane and retinyl propionate (RP) under the same concentration conditions. Its collagen-promoting activity at the same concentration is 4 times of that of Pro-Xylane (2 mg/mL), and is 3-4 times of that of retinyl propionate (3 mg/mL, 10 mg/mL). In addition, it can also be observed in the transcriptome analysis results of the HDF cells (FIG. 8B) that oligo-CA has transcription up-regulatory effect on many other types of collagens, including type 4, type 6, type 7, type 9, type 17, type 22, type 25, and the like. These results show that oligo acid has a good application prospect in the aspect of promoting collagen activity.

### Example 7

According to the experimental method of light damage in the experimental method, the phototoxicity of oligo colanic acid was tested, and the results are shown in FIG. 9. FIG. 9 shows that no significant cytotoxicity was observed in HDF cells irradiated after the addition of oligo colanic acid CA (cell viability > 90% can be considered non-toxic); on the contrary, as the addition amount of oligo colanic acid is increased, the protection effect on cells is enhanced, from 90% at 2 mg/mL to 97% at 10 mg/mL. However, retinyl propionate had significant cytotoxicity both at 3 mg/mL and 10 mg/mL.

### Example 8

According to the method for anti-wrinkle and collagen-promoting under light irradiation conditions in the experimental method, the activity of oligo colanic acid to promote the synthesis of type I collagen and type III collagen by HDF cells under light irradiation conditions, and the results are shown in FIG. 10.

FIG. 10 shows that oligo colanic acid shows stronger activity in promoting type I and type III collagen in light-exposed HDF cell model. Compared with Pro-Xylane, at a concentration of 2 mg/mL, oligo colanic acid promoted type I collagen is 2.3 times of that of Pro-Xylane, and type III collagen is 2.8 times of that of Pro-Xylane. Compared with retinyl propionate, under the same concentration, oligo colanic acid promoted type I collagen is 18 times (3 mg/mL) of that of retinyl propionate and 2 times (10 mg/mL) of that of retinyl propionate, and type III collagen is 10 times (3 mg/mL) and 28 times (10 mg/mL) of that of retinyl propionate. It can be seen that oligo colanic acid is not only non-phototoxic, but also has both the activity of promoting the type I and the type III collagen, and the efficacy thereof is significantly higher than that of Pro-Xylane and retinyl propionate.

### Example 9

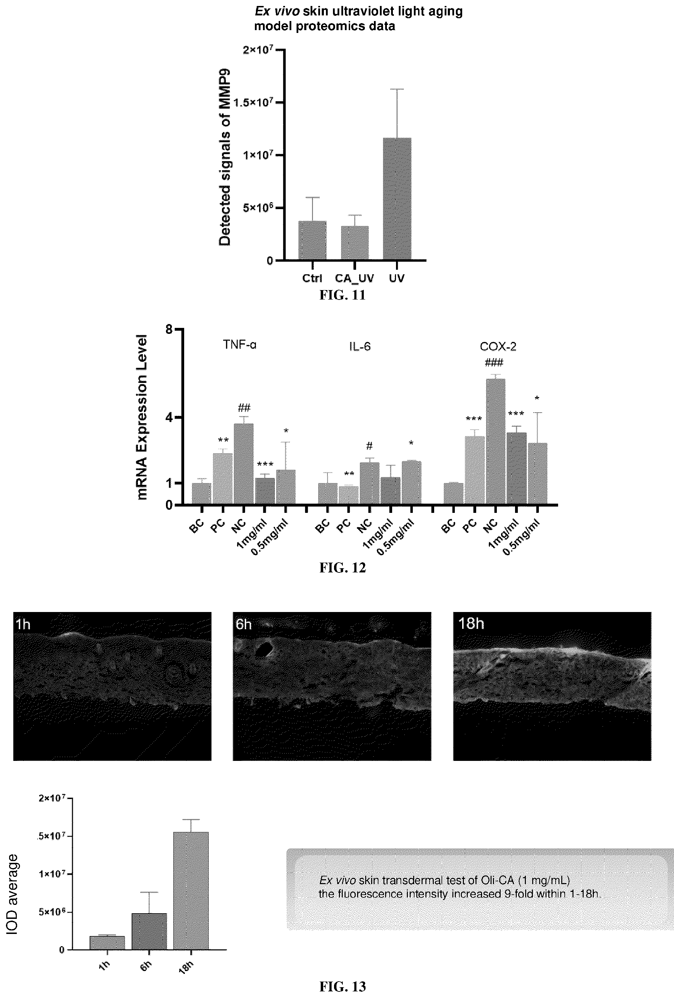

The MMP9 inhibitory effect of the oligo colanic acid on the skin model irradiated by ultraviolet light was tested to obtain proteomic data of *ex vivo* skin under ultraviolet light irradiation conditions. It can be observed from FIG. 11 that the content of MMP9 of the group with the addition of colanic acid was significantly decreased, and MMP9 is a key target for inhibiting the DEJ aging. The increase of MMP9 skin content results in rapid loss of type VI collagen of the DEJ layer, thereby causing the DEJ to become smooth. The DEJ layer is the connecting layer of the dermis layer and epidermis layer, the smoother the DEJ layer, the less contact between the dermis and epidermis layers, and the more aging will occur.

### Example 10

According to the cell culturing and modeling methods in the experimental method of inflammation (keratinocytes) in the experimental method, TNF-α, IL-6 and COX-2 (ELSIA kits: CSB- E04740h, CSB-E04638h, CSB- E10103h from CUSABIO) were tested, thereby testing the anti-inflammatory effect of oligo colanic acid on human keratinocytes. FIG. 12 shows that oligo colanic acid has a good anti-inflammatory effect on human keratinocytes, and can significantly inhibit the expression of TNF-α, IL-6 and COX-2 at a condition of 1 mg/mL.

### Example 11

According to the transdermal experiment method in the experimental method, the transdermal property of oligo colanic acid is tested. FIG. 13 shows that after the oligo colanic acid was fluorescently labeled, it can be observed that the transdermal amount of the molecule within 18 h was significantly increased by observing the transdermal test of *ex vivo* skin, and the fluorescence intensity had a 9-fold enhancement. At the same time, it can be observed that the molecules can effectively penetrate into the dermis layer.

The positioning of oligo colanic acid within cells was tested by the following method.

Cell Culture: HDF cells were cultured in DMEM complete medium (DMEM + 10% FBS + 1% P/S), placed in an incubator for static culturing at 37 °C, 5% CO₂.

Sample Treatment: 1 mg/mL of CA powder with fluorescence labeling was dissolved in the culture medium, and the solution was filtered and sterilized with a 0.2 µm sterile filter membrane after complete dissolving.

Experimental Treatment: HDF cells were inoculated into a 96-well plate, and a sample group, a blank control group and a positive control group were set. At least 3 duplicate wells were set for each tested indicator per sample. After culturing for 24-48h, the cell supernatant was removed, DAPI and Mitotracker staining solution were added to stain the nucleus and mitochondria, respectively, the plate was washed two to three times with PBS after each staining, and finally fluorescence was observed with an optical microscope.

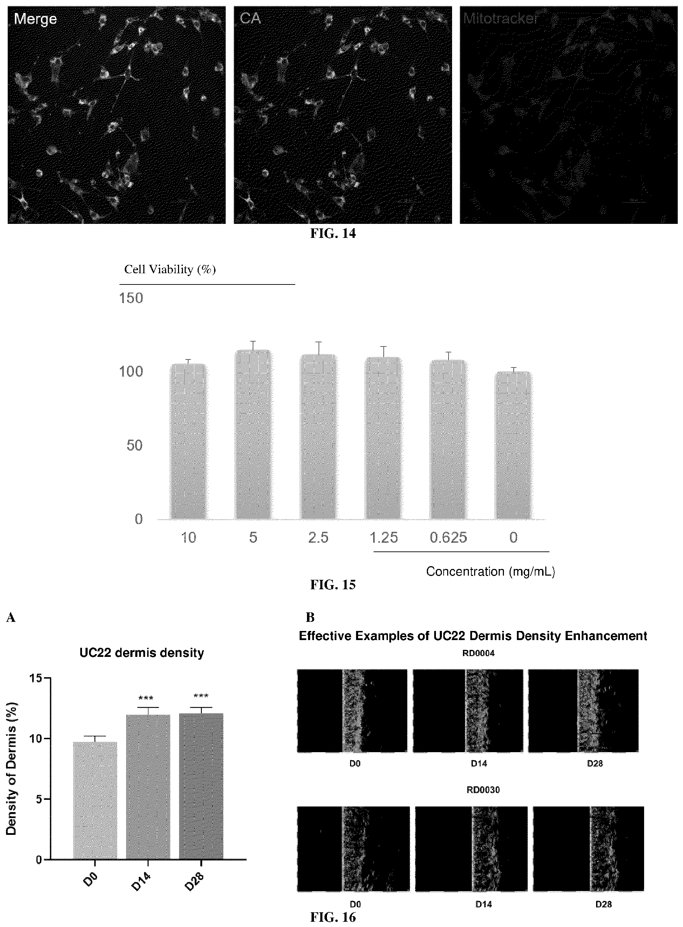

FIG. 14 shows that oligo colanic acid can effectively access into the cell interior, and by mitochondria staining of mitotracker, it can be observed that oligo colanic acid (green) and mitochondria (red) are overlapped, so that it can be seen that oligo colanic acid can penetrate through the cell membrane and enter the mitochondria. On the contrary, the colanic acid and the nucleus (blue) did not show the overlapping phenomenon, and therefore, we hold that the oligo colanic acid is less likely to enter the nucleus.

In addition, the cytotoxicity test results of oligo colanic acid (FIG. 15) shows no cytotoxicity up to a concentration of 10 mg/mL.

### Example 12: Human Experiment Test

Human experiments of oligo colanic acid sodium salt were performed as follows, and the results are shown in FIGs. 16-25.

Sixty-six healthy Chinese male/female subjects were selected and randomly divided into two groups of 33 persons, with an age range of 30 to 60 years. The subject has dry and flabby face, lack of elasticity, and a skin elasticity R2 between 0.35 and 0.55; facial fine lines or wrinkles, with a wrinkle grade of eye corner wrinkles of 2 to 5; and weak skin barrier and high sensitivity. Using pre- and post-control and between-group control methods, the skin stratum corneum moisture content, skin transepidermal water loss value, skin elasticity, skin firmness, dermal density, dermal thickness, and full skin thickness of the subjects were measured for subjective assessment, and localized and full-face photographs were taken. The test results were compared by a statistical test method to determine whether they were statistically different.

Detected Parts
(1) Skin stratum corneum moisture content - cheek
(2) Skin transepidermal water loss value - cheek
(3) Skin elasticity R2 - cheek
(4) Skin firmness F4 - cheek
(5) UC22 dermal density - cheek
(6) UC22 dermal thickness - cheek
(7) UC22 full skin thickness - cheek
(8) PRIMOS CR under eye wrinkle area - under eye
(9) PRIMOS CR under eye wrinkle volume - under eye
(10) PRIMOS CR cheek roughness Ra - cheek
(11) VISIA CR skin color L value - full-face photographing and analysis of cheek
(12) VISIA CR skin color b value - full-face photographing and analysis of cheek
(13) VISIA CR skin color ITA° value - full-face photographing and analysis of cheek
(14) VISIA CR skin transparency parameter - full face
(15) VISIA CR skin gloss parameter - full-face photographing and analysis of cheek
(16) VISIA CR skin red area percentage - full-face photographing and analysis of cheek
(17) VISIA CR eye corner wrinkle area percentage - full-face photographing and analysis of eye corner
(18) VISIA CR pore area percentage - full-face photographing and analysis of cheek
(19) VISIA CR jawline angle - full-face photographing and analysis of lower jaw
(20) Epidermal dermal junction DEJ layer standardized area DEJI - eye corner
(21) Aging index ELCOR - eye corner
(22) Aging index SAAID - eye corner
(23) Epidermis layer thickness TED - eye corner
(24) Elastic fiber fluorescence intensity - eye corner
(25) Collagen fiber harmonic intensity - eye corner
(26) Eye corner wrinkle grade assessment - eye corner
(27) Under eye wrinkle grade assessment - under eye
(28) Gloss score - cheek
(29) Elasticity score - cheek
(30) Fineness score - cheek
(31) Smoothness score - cheek
(32) Self-evaluation - full face

### Day 0:

Subjects visited and were assessed by a technician for facial skin condition, and those with dry skin or in wrinkle and redness conditions were selected among the subjects who participated in the trial;
Cleaning the Face and Waiting: Subjects cleaned their face with a cleansing product and dried their skin with a dry facial tissue, and sat still for 20 min in the laboratory at a temperature of 21°C ± 1°C and a humidity of 50% RH ± 10% RH;
Data Collection of Basic Skin Values of the Test Subjects: The laboratory technician operated SUPERVISION 780, VISIA CR, Cutometer, PRIMOSCR, Corneometer, Vapometer, UC22 instruments to measure the basal values of the relevant indexes of the test areas of the subjects;
Professional rating and recording of eye wrinkles of the subjects were conducted by a trained dermatologist;
Skin gloss, fineness, smoothness, and elasticity of the subjects were scored and recorded by a professional evaluator;
The laboratory technician instructed the subjects to use the product according to the product use requirements and provided written test precautions and instructions for product use. According to the randomization table, the subjects were randomly divided into two groups. One person in each group was randomly selected to use the product on half of his/her face, and a multi-point moisture test was conducted on the face 2 h and 4 h after using the product.

Subjects received the product and left the laboratory.

### Day 14:

Subject return visit, cleaning the face and waiting: subjects cleaned their face with a cleansing product and dried their skin with a dry facial tissue, sat still for 20 min in the laboratory at a temperature of 21°C ± 1°C and a humidity of 50% RH ± 10% RH, and completed a D14 self-assessment questionnaire during the 20 min period of sitting;
Skin D14 Data Collection of Test Subject: The laboratory technician operated SUPERVISION 780, VISIA CR, Cutometer, PRIMOSCR, Corneometer, Vapometer, UC22 instruments to measure the relevant indexes of the test areas of the subjects;
Professional rating and recording of eye wrinkles of the subjects were conducted by a trained dermatologist;
Skin gloss, fineness, smoothness, and elasticity of the subjects were scored and recorded by a professional evaluator;
Staff checked the subject's usage records and products;
Subjects left the laboratory.

### Day 28:

Subject return visit, cleaning the face and waiting: subjects cleaned their face with a cleansing product and dried their skin with a dry facial tissue, sat still for 20 min in the laboratory at a temperature of 21°C ± 1°C and a humidity of 50% RH ± 10% RH, and completed a D28 self-assessment questionnaire during the 20 min period of sitting;
Skin D28 Data Collection of Test Subject: The Laboratory technician operated SUPERVISION 780, VISIA CR, Cutometer, PRIMOSCR, Corneometer, Vapometer, UC22 instruments to measure the relevant indexes of the test areas of the subjects;
Professional rating and recording of eye wrinkles of the subjects were conducted by a trained dermatologist;
Skin gloss, fineness, smoothness, and elasticity of the subjects were scored and recorded by a professional evaluator;
Staff checked and collected the subject's usage records and products;
Subjects left the laboratory;
The test process ended.

FIG. 16 shows the effect of oligo colanic acid sodium salt on improving the density of human dermis layer. As can be seen from FIG. 16, the density of dermis UC22 increased by 22.87% after the subjects continuously used the essence containing 0.5% (w/v) oligo colanic acid sodium salt for 14 days; and increased by 24.00% after continuously used for 28 days.

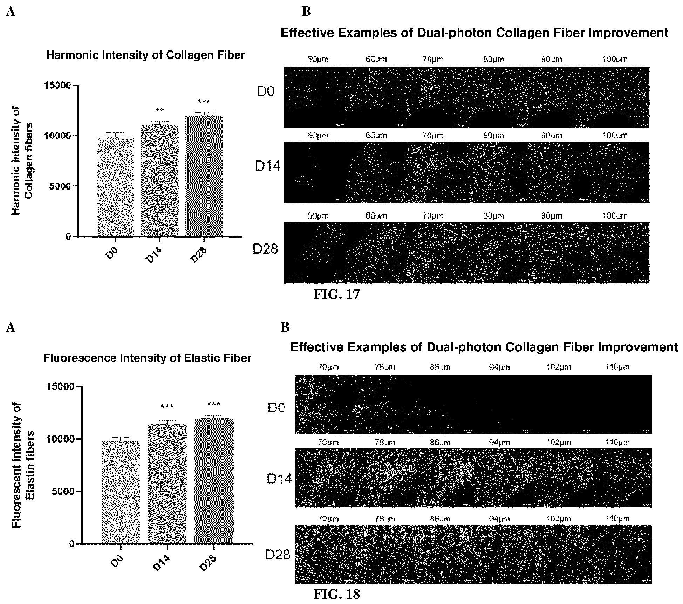

FIG. 17 shows the effect of oligo colanic acid sodium salt on improving the expression of collagen in human dermis layer. As can be seen from FIG. 17, the harmonic intensity of the collagen fiber increased by 12.31% after the subjects continuously used the essence containing 0.5% (w/v) oligo colanic acid sodium salt for 14 days, and increased by 21.61% after continuously used for 28 days.

FIG. 18 shows the effect of oligo colanic acid sodium salt on improving the expression of elastic protein in human dermis layer. As can be seen from FIG. 18, the fluorescence intensity of the elastic fibers increased by 17.02% after the subjects continuously used the essence containing 0.5% (w/v) oligo colanic acid sodium salt for 14 days, and increased by 22.13% after continuously used for 28 days.

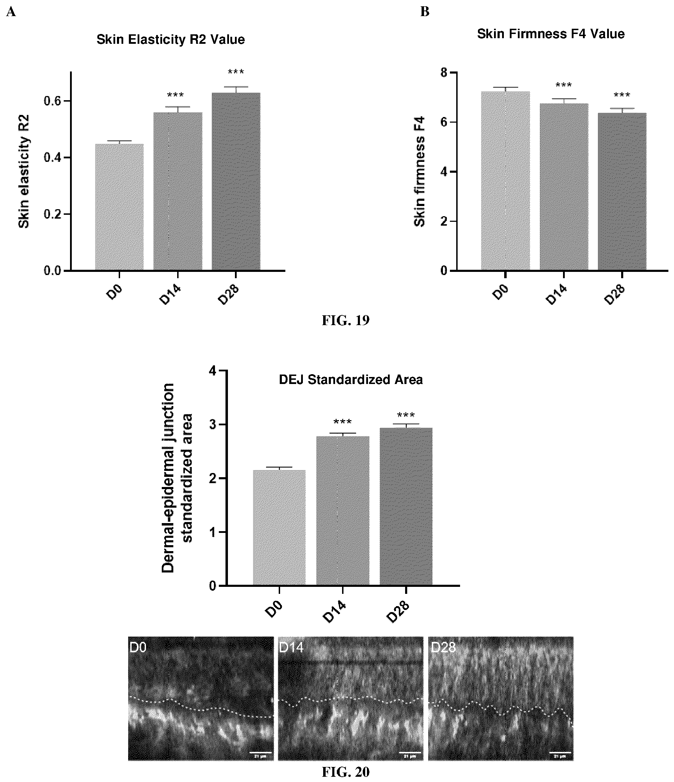

FIG. 19 shows the effect of oligo colanic acid sodium salt on improving the human skin elasticity and skin refinement. As can be seen from FIG. 19, skin elasticity R2 increased by 24.44%, and skin firmness F4 decreased by 6.62% after the subjects continuously used the essence containing 0.5% (w/v) oligo colanic acid sodium salt for 14 days; and R2 increased by 40.00%, and F4 decreased by 12.00% after continuously used for 28 days.

FIG. 20 shows the effect of oligo colanic acid sodium salt on improving the DEJ normalized area of human skin. As can be seen from FIG. 20, the dermal epidermal junction DEJ layer normalized area DEJI increased by 28.70% after the subjects continuously used the essence containing 0.5% (w/v) oligo colanic acid sodium salt for 14 days; and increased by 36.11% after continuously used for 28 days.

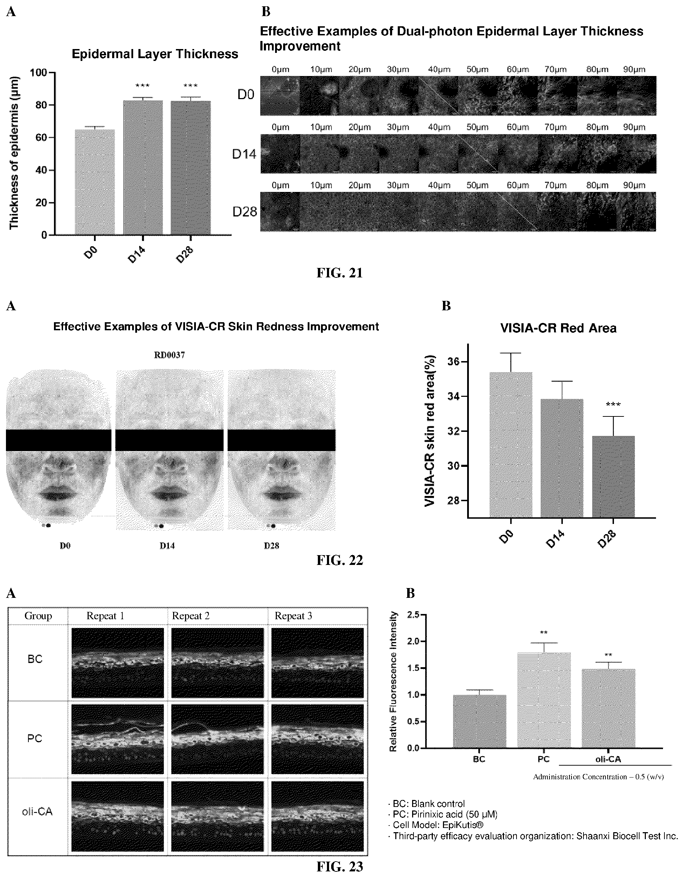

FIG. 21 shows the effect of oligo colanic acid sodium salt on improving the human skin epidermis layer thickness. As can be seen from FIG. 21, the increase rate of epidermis layer thickness was 27.78% after the subjects continuously used the essence containing 0.5% (w/v) oligo colanic acid sodium salt for 14 days, and the increase rate was 27.29% after continuously used for 28 days.

FIG. 22 shows the effect of oligo colanic acid sodium salt on improving the skin redness of human skin. As can be seen from FIG. 22, VISIA-CR skin red zone area percentage decreased by 10.45% after the subjects continuously used the essence containing 0.5% (w/v) oligo colanic acid for 28 days.

FIG. 23 shows the effect of oligo colanic acid sodium salt on improving the 3D skin filaggrin (FLG) content. As can be seen from the test results of the 3D skin experiment in FIG. 23, as compared with the BC control group, oligo colanic acid sodium salt at the concentration of 0.5% (w/v) significantly increased the filaggrin (FLG) content, with an improvement rate of 49.00%.

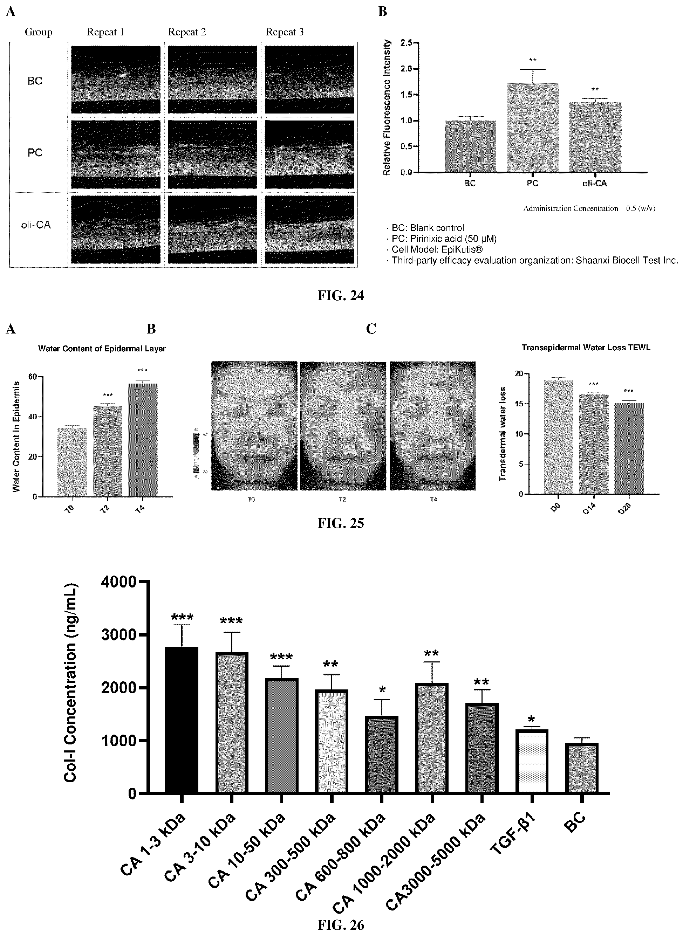

FIG. 24 shows the effect of oligo colanic acid sodium salt on improving the 3D skin aquaporin 3 (AQP3) content. As can be seen from the test results of the 3D skin experiment in FIG. 24, as compared with the BC control group, oligo colanic acid sodium salt at the concentration of 0.5% (w/v) significantly increased the aquaporin 3 (AQP3) content, with an improvement rate of 37.00%.

FIG. 25 shows the effect of oligo colanic acid sodium salt on the water retention and moisturization of human skin. As can be seen from FIG. 25, the skin moisture content of the stratum corneum skin was significantly increased by 31.76% after the subjects continuously used the essence containing 0.5% (w/v) oligo colanic acid sodium salt for 14 days; and increased by 63.84% after continuously used for 28 days. The skin transepidermal water loss value significantly decreased by 12.57% compared with the basal value; and decreased by 20.08% after continuously used for 28 days.

### Example 13

According to the preparation method, colanic acid sodium salts with the molecular weight of 1-10 kDa, 1-8 kDa, 1-6 kDa, 1-5 kDa, 1-4 kDa, 1-2 kDa, 2-10 kDa, 2-8 kDa, 2-6 kDa, 2-4 kDa, 2-3 kDa, 3-6 kDa, 4-10 kDa, 4-8 kDa, 4-6 kDa, 5-10 kDa, 5-8 kDa, 6-10 kDa, or 6-8 kDa were prepared and tested according to the same method of Examples 1 to 12.

### Example 14

According to the anti-wrinkle (collagen promoting) method in the experimental method, the effect of different molecular weights of colanic acid sodium salt (CA) on stimulating the expression of type I collagen in human fibroblasts at a concentration of 3 mg/mL were tested, and the results are shown in FIG. 26. As can be seen from FIG. 26, oligo colanic acid sodium salt with molecular weights in a range of 1-3 kDa and 3-10 kDa have better anti-aging and collagen-promoting activity.

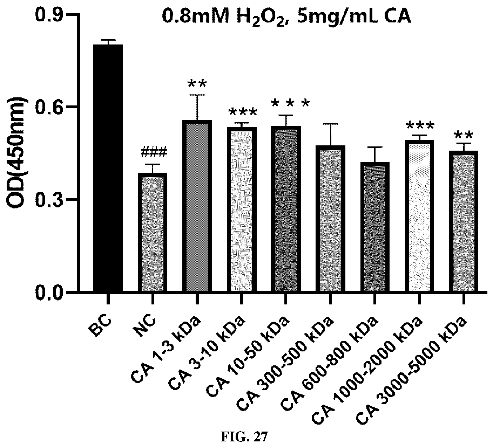

According to the experimental method of cell viability-CCK8, the skin protection effect of colanic acid sodium salts (CA) with different molecular weights were tested, and the results are shown in FIG. 27. As can be seen in FIG. 27, oligo colanic acid sodium salt with molecular weights in a range of 1-3 kDa and 3-10 kDa have better skin protective effects.

### INCORPORATION BY REFERENCE

The entire contents of each patent and scientific literature referred to herein are incorporated herein by reference for all purposes.

### EQUIVALENCE

The present invention may be embodied in other specific ways without departing from its spirit or essential feature thereof. Accordingly, the foregoing embodiments should in all cases be taken as illustrative and not as limitations of the invention described herein. Accordingly, the scope of the present invention is indicated by the accompanying claims and not by the foregoing description, and is intended to cover all variations within the meaning and scope of equivalence of the claims.

## Claims

1. Use of a colanic acid or a physiologically acceptable salt thereof, or a composition comprising a colanic acid or a physiologically acceptable salt thereof in skin anti-aging and/or skin protection, **characterized in that** the molecular weight of the colanic acid or the physiologically acceptable salt thereof is in a range of **1-10** kDa, preferably in a range of 1-3 kDa or 3-10 kDa.

2. The use of claim 1, wherein the skin anti-aging comprises improving collagen content in skin, preferably type I and type III collagen content in skin.

3. The use of claim 1, wherein the skin protection comprises enhancing protection function of skin cells, such as increasing viability of skin cells.

4. The use of claim 1, wherein the skin anti-aging and the skin protection comprise improving condition or appearance of skin.

5. The use of any one of claims 1-4, wherein the content of the colanic acid or the physiologically acceptable salt thereof is 0.01-10% (w/v), preferably 0.1-5% (w/v), more preferably 0.5-3% (w/v).

6. The use of any one of claims 1-5, wherein the physiologically acceptable salt of colanic acid is colanic acid sodium salt.

7. The composition of any one of claims 1-6, wherein the colanic acid or the physiologically acceptable salt thereof, or the composition comprising the colanic acid or the physiologically acceptable salt thereof, is formulated for topical, oral, intramuscular, subcutaneous or intravenous administration.

8. The composition of any one of claims 1-7, wherein the composition comprising the colanic acid or the physiologically acceptable salt thereof further comprises a cosmetically or pharmaceutically acceptable additive.

9. A composition, **characterized in that** the composition comprises a colanic acid or a physiologically acceptable salt thereof as an active ingredient, and the molecular weight of the colanic acid or the physiologically acceptable salt thereof is in a range of 1-10 kDa, preferably in a range of 1-3 kDa or 3-10 kDa.

10. The composition of claim 9, wherein the content of the colanic acid or the physiologically acceptable salt thereof in the composition is 0.01-10% (w/v), preferably 0.1-5% (w/v), more preferably 0.5-3% (w/v).

11. The composition of claim 9 or 10, wherein the composition comprises merely the colanic acid or the physiologically acceptable salt thereof as the active ingredient.

12. The composition of any one of claims 9-11, wherein the composition is formulated for topical, oral, intramuscular, subcutaneous or intravenous administration.

13. The composition of any one of claims 9-12, wherein the composition further comprises a cosmetically or pharmaceutically acceptable additive.

14. The composition of any one of claims 9-13, wherein the physiologically acceptable salt of colanic acid is colanic acid sodium salt.
